# EUROPEAN PATENT SPECIFICATION

(11) **EP 1 453 528 B1**
(45) Date of publication and mention of the grant of the patent: **22.07.2009**
(21) Application number: 02781641.2
(22) Date of filing: 14.12.2002
(51) Int. Cl.: A61K 36/78, A61K 31/12, A61K 31/121, C07C 49/203, C07C 49/647

(54) **COMPOSITIONS CONTAINING CURCUMA EXTRACTS FOR THE TREATMENT OF NEUROCEREBROVASCULAR DISORDERS**
ZUSAMMENSETZUNGEN AUS CURCUMA EXTRAKTEN FÜR DIE BEHANDLUNG VON NEUROZEREBROVASKULÄREN ERKRANKUNGEN
COMPOSITION CONTENANT EXTRAITS DE CURCUMA DESTINÉE AU TRAITEMENT DE TROUBLES NEUROCEREBROVASCULAIRES

(30) Priority: 14.12.2001 US 340165 P
(43) Date of publication of application: 08.09.2004
(73) Proprietor: Council of Scientific and Industrial Research, New Delhi 110 001 (IN)
(72) Inventor: RAY, Madhur, Central Drug Research Institute, 226 001 Lucknow (Uttar Pradesh) (IN); PAL, Raghwendra, Central Drug Research Institute, 226 001 Lucknow (Uttar Pradesh) (IN); SINGH, Satyawan, Central Drug Research Institute, 226 001 Lucknow (Uttar Pradesh) (IN); KHANNA, Nandoo M., Central Drug Res. Institute, 226 001 Lucknow (Uttar Pradesh) (IN)
(74) Representative: Fairbairn, Angus Chisholm
(86) International application number: PCT/IB2002/005366
(87) International publication number: WO 2003/051380

(56) References cited:
- EP-A- 0 440 885
- WO-A-01/30335
- WO-A-02/074295
- DE-A- 10 029 770
- US-A- 4 842 859
- DATABASE WPI Derwent Publications Ltd., London, GB; AN 1997-245838 XP002236056 & CN 1 100 321 A (YE QIZHI (CN)), 22 March 1995 (1995-03-22)
- AMMON H P T ET AL: "PHARMACOLOGY OF CURCUMA LONGA" PLANTA MEDICA, THIEME, STUTTGART, DE, vol. 57, 1991, pages 1-7, XP000916128 ISSN: 0032-0943
- SRIVASTAVA K C: "EXTRACTS FROM TWO FREQUENTLY CONSUMED SPICES - CUMIN (CUMINUM CYMINUM) AND TURMERIC (CURCUMA LONGA) - INHIBIT PLATELET AGGREGATION AND ALTER EICOSANOID BIOSYNTHESIS IN HUMAN BLOOD PLATELETS" PROSTAGLANDINS LEUKOTRIENES AND ESSENTIAL FATTY ACIDS, CHURCHILL LIVINGSTONE MEDICAL JOURNALS,, GB, vol. 37, 1989, pages 57-64, XP000934146 ISSN: 0952-3278
- DATABASE CA [Online] CHEMICAL ABSTRACTS SERVICE, COLUMBUS, OHIO, US; JAYADEEP, V. R. ET AL: "Changes in the prostaglandin levels in alcohol toxicity: effect of curcumin and N-acetylcysteine" retrieved from STN Database accession no. 134:158763 XP002236053 & JOURNAL OF NUTRITIONAL BIOCHEMISTRY (2000), 11(10), 509-514,
- DATABASE CA [Online] CHEMICAL ABSTRACTS SERVICE, COLUMBUS, OHIO, US; RAJAKRISHNAN, V. ET AL: "Neuroprotective role of curcumin from Curcuma longa in ethanol-induced brain damage" retrieved from STN Database accession no. 132:246307 XP002236054 & PHYTOTHERAPY RESEARCH (1999), 13(7), 571-574,
- DATABASE CA [Online] CHEMICAL ABSTRACTS SERVICE, COLUMBUS, OHIO, US; YAMAMOTO, HISANORI ET AL: "Inhibitory effect of curcumin on mammalian phospholipase D activity" retrieved from STN Database accession no. 128:57137 XP002236055 & FEBS LETTERS (1997), 417(2), 196-198,
- DATABASE WPI Derwent Publications Ltd., London, GB; AN 2002-263510 XP002236057 & JP 2002 030081 A (SHISEIDO CO LTD), 29 January 2002 (2002-01-29)

## Description

The present invention relates to a composition obtained from a lipid soluble extract from rhizomes and/or leaves of *Curcuma* species of the zingiberaceae family, and also use of the said composition, for the treatment of neurocerebrovascular disorders.

Neurocerebrovascular diseases like cerebrovascular infarction, stroke, ischemic attacks etc. are caused by an interruption of the blood supply resulting from disease of the arteries carrying blood to the brain.

Of the three general types of stroke, cerebral hemorrhage is caused by rupture of a blood vessel with bleeding into the brain (intra cerebral hemorrhage) or under its covering membrane, while cerebral thrombosis stems from obstruction of a cerebral blood vessel when a blood clot forms within the walls.

The clot may be caused by abnormal thickening of the blood, damage to the vessel wall from arteriosclerosis, atherosclerosis, inflammation of the arteries or inflammation of the veins.

If the blood supply is stopped completely or is reduced to less than one-fourth its normal level, softening of the brain (cerebral infarction) results, causing permanent brain damage.

Cerebral embolism is obstruction of a cerebral artery by a blood clot or a foreign body migrating from another part of the body' circulation like when a clot that has formed on the inside wall of one of the arteries in the neck travels up to the brain and blocks a major artery branch.

Transient ischemic attacks (TIAs) are brief episodes of symptoms caused by temporary interruptions of the blood supply. Reversible ischemic neurological deficits( RINDs) are small cerebral infarction. Multiple cerebral infarction can lead to permanent confusion and memory loss. Ischemic stroke is a medical emergency. After TIAs or stroke occur, treatment may be surgical or medical.Surgery may be needed in some cases to remove any blockage of blood vessels going to the brain.

Medication can prevent the formation of blood clots on the atherosclerotic plaques within the vessel wall. Brain swelling commonly accompanies brain infarction or hemorrhage. No satisfactory treatment is available.

Currently used drugs in peripheral vascular and cerebral disorders include ergot alkaloids, aspirin, anti-coagulants etc. The latter are used following strokes to prevent further cerebrovascular incidents but their use is contraindicated if the stroke was the result of hemorrhage.

The use of TICLOPIDINE, a highly effective anti-platelet agent to treat stroke cases is restricted in its long term use due to its adverse side-effects. Tissue plasminogen activator (t-PA) used to treat clots in the coronary arteries (acute heart attack), is a natural clot dissolving substance produced by the body which can blow open a blood clot in the brain that causes the acute ischemic brain damage characteristic of a stroke. While t-PA can dissolve the blood clot that causes a blood vessel blockage, there are other complications which occur during ischemic stroke which must be addressed if permanent brain damage is to be prevented. It is critically important to have nitric oxide (NO) and superoxide scavengers in the blood stream when t-PA is administered to reduce the free radical damage that will occur when the blood flow is restricted and even more when the flow is resumed.

Nitric oxide(NO) and superoxides inflict damage on important biomolecules and their increased production has been implicated in human diseases like cerebro-,cardiovascular, inflammatory, neurological dysfunctions and cancer etc.[ Onoda M., Inano H., Nitro oxide:Biology and Chemistry, 4,(5),505-515 (2000)].

Most strokes culminate in a core area of cell death (infarction) and the blood flow is so drastically reduced that the cells usually can not recover.Brain cells die as a result of the action: calcium.activated proteases (enzymes which digest cell proteins), lipases (enzymes which digest cell membranes) and free radicals formed as a result of the ischemic cascade. Without neuroprotective agents, nerve cells may be irreversibly damaged within several minutes. Any disruption of blood flow to the brain causes massive free radical damage that induces much of the re-perfusion injury to brain cells, typical of stroke. When blood flow is interrupted and subsequently restored (reperfused), tissues release iron that acts as a catalyst for the formation of free radicals that often permanently damage brain cells. Protecting brain cells from injury caused by blood flow disruption, therefore, is of prime importance. If an ischemic stroke is happening. , the use. of large quantities of anti-oxidants like melatonin, vitamins and herbs like *Ginkgo biloba* have been suggested to provide some benefit. Magnesium in an oral dose of 1500 mg. is a safe nutrient to relieve an arterial spasm, a common problem in thrombotic strokes.

The ancient Indian system of Ayurveda medicine is concerned with the prevention, diagnosis and cure of disease. The word "dis-ease"- a right translation of illness is viewed as a dysfunction of the whole body and is attributed to the circulation and transformation of ubiquitous humoral fluids.

Most of the Ayurvedic drugs are products of high repute which act on a number of dysfunctions of the body involving various organs and aim at preventing problems or restoring a normal situation, and try to recover the patient completely. Evolved over a long period of time and experimentation, they are the results of a particular combination of certain _ fundamental elements which determine their properties which in turn are responsible for the chemical biological or therapeutic effects of those substances. There is no substance when correctly prepared which can not be used as remedy.

Ayurveda describes a number of beneficial effects of rhizomes and leaves of various species belonging to zingiberaceae family, especially those of *Curcuma longa* L.syn.*Curcuma domestica* Valeton, rhizomes and leaves popularly known as *Turmeric* or *Haldi.* Prominent among these are the anti-bacterial, antifungal wound healing and the anti-inflammatory actions which enabled *turmeric* paste to be used as a household remedy to treat wounds and inflammation.

In recent years, its constituents-Curcumin and other curcuminoids have been found to exhibit besides these activities, choleretic, cholagogic, anti-oxidant, anti-cancer, inhibition of leukotriene biosynthesis, 5-lipoxygenase, cyclo-oxygenase, lipid peroxidation, superoxide and nitric oxide (NO) scavenging effects. *Turmeric,* a highly reputed herb in Indian system of Ayurveda medicine, is the rhizome of *Curcuma longa* L.Syn. *Curcuma domestica* Valeton (Fam. Zingiberaceae) ,which grows abundantly in India. It has long been used a spice and a colouring agent in food as well as a naturally occurring medicine. Its powder or extracts are recommenced to treat wounds and inflammation.

A major constituent Curcumin was developed as an anti-inflammatory agent [Srimal RC., Khanna N.M.,Dhawan B.N., Ind. J. Pharmacol.,3,10 (1971)]. Other therapeutic properties of Curcumin, various curcuminoids and some other constituents of *Curcuma* species include anti-bacterial, anti-fungal [Schraufstatter F., Brent H., Nature, 164,456(1949), Arch.Dermatol..u.Syphilis,188,250 (1949);Lutomski.J.,RedziaB.,Debska W,Planta Med., 26, 9 (1974) ; Rao B.G.N., Joseph P., Reichst,Aromen Koerperflegem, 21,405-406(1971); Swada T., Yamahara J., Shimazu S., Ohta T., Shoyakugaku Zasshi, *2*,11-16(191), Prasad C. R., Sirsi M., J.Sci. Ind. Res.,C. 15, 239-41(1957) ; Schraufstatter E., Deutsh. S. Z. Naturforsch. 4,276 (1949) ; Chopra R. N., Gupta J. C., Chopra G. S.,Ind. J. Med. Res., 29, 769-72 (1941)], anti-oxidant [Ramaswamy T. S., Banerjee B. N., Ann. Biochem. Exp. Med, 8, 55 (1948) ; Chipault .J. R., Mizuno G. R., Lundberg W. O., Food Res., 10, 209, (1956 )]; inhibition of lipid per-Oxidation [Sharma S.C., Mukhtar H.., Sharma S. K., Krishnamurty C. R., Biochem. Biopharmacol., 21, 1210-14 ( 1972 ); Zu S., Tang. X. Lin Y., Zhougcuoyev., 22, 264-5(1991); Sharma O. P., Biochem. Biopharmacol. 25 , 1811(1976 )]; active oxygen species scavenging and prevention of increased free radical formation by Curcumin in the body [Tennesen H.H.,Inter.J. Pharmacol., 50, 67-69(1989), Kunchandy E.., Rao M.N. A., Inter. J. Pharmacol., 58, 237 (1990)]; inhibitory activity for iNOS induction by lipopolysaccharide in the mammary gland and scavenging activity for NO radicals by Curcumin, [ Onoda M., Inano H., Nitric Oxide : Biology and Chemistry, 4 ,505-515 ('2000)], anti-inflammatory [Arora R.B., Basu N., Kapoor V., Jain A., Proc. Second Indo Soviet Symposium on Natural Products, New Delhi,1970, p. 170., Ind. J.Med. Res., 59 ,10 (1971); Mukhopadhya A., Basu N., Ghatak N., Singh K. P., Gujral P.D., Proc. Int. Union of Physiol. Sci., 11,241 (1974); Ghatak N.N., Basu N., Ind. J. Exp. Bio, 10,235 (1972), Chandra D., Gupta S.S., Ind. J. Med. Res., 60, 138-142 (1972)]; anti-cancer [Soudamini K.K., Kuttan R., J. Ethnopharmacol. 27,227 (1989);Kuttan R. Bhanumatty P., Nirmala K., George M.C., Cancer Lett., 29, 197 (1985)]; antioxidant and antitumor promotor which induces apoptosis in human leukemia cells[Rao M.L., Huang T.S., Lin J.K., Biochem. Biophysic. Acta, 1817, 98-100(1996)], inhibition of cell growth in chinese hamster ovary cell culture and cytotoxicity to lymphocytes and Dalton's lymphomaCells., [ Cancer Lett: (Ireland), 29,197 (1985) via Chem. Abstr. 104, 61654 d (1986)], tumor protecting activity in mouse skin carcinogenesis induced by 7,12-dimethyl benz (a) anthracene[Kyoto-Furiton Doigaku Zasshi, 96, 725 (1987)-via Chem. Abstr., 107, 211555a (1987)], inhibition of HIV protease [ Suz Luz, Craik-C.S.,Oritz T., Montanello P.R., Proc. 205, ACS National Meeting, Denver, colarado, 28 March-2 april, Amer. Chem. Soc. Med. Chem. Div.(1993), Take Y., Inoyya H., Nakamura S., Alauddin H.S., Kuba A., J.Antibiot., 42,107-118 (1989)], inhibition of lipoxygenase, cyclooxygenase [ Tennesen. H.H., Int. J Pharmacol., 50.,67 (1989), inhibition of ADP-epinephrine and collagen induced platelet aggregation, [Srivastava. R., Puri V., Srimal R.C., Dhawn B.N.; Arznei Forsch.,*36*, 715-717(1986)]; protection against thrombotic challenge [ Srivastava R., Dixit M., Srimal R.C.,Dhawan B.N.,Thromb. Res.,40, 413-17(1985)]; reduction in ratio of total cholesterol / phospholipids in hyperlipidemic rats and elivated HDL-cholesterol and total cholesterol ratio[Ind. J.Physiol.Pharmacol.,32,299 (1988)]; anticoagulent activity [Chem. Pharm. Bull., 33, 1499 (1985)]; inhibition of platelet aggregation, metabolic disorders and hyperlipidemia [Lin Y.,U.S. Patent, 4842849; Chem. Abstr.,111,160200 (1984); Khanna N.M., Sarin J.P.S., Singh S., Pal R., Seth R.K., Nitya Nand S., Indian Patent 162441(1984)]; which makes it useful to prevent cardiovascular disorders like ischemic heart attacks, myocardial infarction etc. In Indo-China region, *Curcuma* extracts are given at parturition on account of their anticoagulent action. Ethyl p-methoxy cinnamate isolated from *Curcuma* rhizomes essential oil exhibit antifungal activity[ Herba Hung., 28, 95(1989), via Chem. Abstr. 111, 191496j (1989)], while furanogermenone and (4S,5S) (+) germacrone 4,5-epoxide also isolated from *Curcuma* rhizomes essential oil exhibits anti-inflammatory and preventive effect against stress ulceration [Yakugaku Zasshi, 106, 1137 (1986),Chem. Abstr..106, 95935c (1987); Zhongyao Tungbto, 10, 134 (1983),Chem. Abstr. 103, 115886d(1985)]. The other reputed herb from Zingiberaceae family, *Zingiber officinale* Rosch, exhibits preventive effects in heart attack or stroke[ Srivastava K.C., Prostaglandins Leukotrienes and Medicines, 13,227-235(1964)]. These and other properties of *Curcuma* extracts and constituents thereof are further disclosed for example in US-A-4,842,859; WO-A-0130335; EP-A-0440885; CN-A-1100321; Ammon H P T et al, Planta Medica, 1991, 57, p1-7; Srivastava K C, Prostaglandins Leukotrienes and Essential Fatty Acids, 1989, 37, p57-64; Jayadeep, V R et al., Journal of Nutritional Biochemistry, 2000, 11 (10), p509-514; Rajakrishnan V et al., Phytotherapy Research, 1999, 13(7), p571-574; and Yamamoto H, et al., FEBS Letters, 1997, 417(2), p196-198.

The main object of the present invention is to provide a composition obtained from a lipid soluble extract from rhizomes and/or leaves of *Curcuma* species, which belong to the Zingiberaceae family, for the treatment of neurocerebrovascular disorders.

The present invention relates to a composition obtained from a lipid soluble extract called *Curcuma* oil. The source of said oil is rhizomes and/or leaves of species of the Zingiberaceae family. The particularly species of the said family used to produce said oil is *Curcuma* species. The said oil is used for the treatment of neurocerebrovascular disorders.

Accordingly the present invention provides a composition obtained from a lipid soluble extract of rhizomes and/or leaves of *Curcuma* species of the Zingiberaceae family, for the treatment of neurocerebrovascular disorders, said composition comprising as its major active constituents ar-d-turmerone of formula 1, and a and β turmerone of formula 2, and optionally further comprising minor constituents selected from curcumene, zingiberine, germacrone, curcumerone, zedoarone, sedoarondiol, isozdedoaronidiol, curcumenone, and curlone, and/or pharmaceutically acceptable additives, as defined in claim 1 herein.

In an embodiment of the present invention, the curcuma species is *Curcuma domestica Valeton.*

In another embodiment of the present invention, ar-d-turmerone constitues 95% of the composition.

In still another embodiment of the present invention, additives are present and selected from melatonin, antioxidant, calcium channel antagonists, tissue plasminogen activator (t-PA), and cell membrane stabilizing agents.

In still another embodiment of the present invention, said composition inhibits nitric oxide synthase (NOS) overproduction, prevents calcium overload in neurons, and scavenges free radicals.

In still another embodiment of the present invention, said neurocerebrovascular disorders are selected from ischaemia, stroke, post- stroke injury, hemorrhage, reperfusion injury, thrombosis, vasoconstriction, nitric oxide-induced free radical oxidative damage, infarction, inflammation, and Alzheimer's disease.

In still another embodiment of the present invention, said composition is in the form of a delivery system selected from tablets, capsules, suppository, beads, and aerosols.

In still another embodiment of the present invention, the use of the composition of claim 1 for treating neurocerebrovascular disorders in animals including humans is provided, by administration of a therapeutically effective amount of the lipid soluble extract.

In still another embodiment of the present invention, said use involves inhibiting nitric oxide synthase (NOS) overproduction, prevention of calcium overload in neurons, and scavenging of free radicals.

In still another embodiment of the present invention, the use is for treating neurocerebrovascular disorders selected from ischaemia, stroke, post-stroke injury, hemorrhage, reperfusion injury, thrombosis, vasoconstriction, nitric oxide-induced free radical oxidative damage, infarction, inflammation, and Alzheimer's disease.

In still another embodiment of the present invention, the use of the composition is in the form of a delivery system selected from tablets, capsules, suppository, beads, and aerosols.

In still another embodiment of the present invention, the use is for treating ischaemia.

In still another embodiment of the present invention, the use is to treat severe brain ischaemia.

In still another embodiment of the present invention, the effective amount for the use ranges between 10-1000 mg/day in a divided dosage schedule.

In still another embodiment of the present invention, the use of the composition is by administration intraperitoneally or post occlusion.

In still another embodiment of the present invention, said use prevents overload of calcium ions in the Mitochondria.

In still another embodiment of the present invention, the use is for treating stroke.

In still another embodiment of the present invention, the use is for treating stroke selected from thrombotic, embolic, and focal stroke.

In still another embodiment of the present invention, the use is for treating hemorrhage.

In still another embodiment of the present invention, the effective amount for the use ranges between 10-500 mg/day in a divided dosage schedule.

In still another embodiment of the present invention, the use is for treating thrombosis.

In still another embodiment of the present invention, the use is for treating thrombosis selected from cerebral, coronary, and deep vein thrombosis.

In still another embodiment of the present invention, the use brings down the thrombus to one-fourth.

In still another embodiment of the present invention, the use is for treating hypertension.

In still another embodiment of the present invention, the use brings down the blood pressure by about 40%.

In still another embodiment of the present invention, the use maintains the blood pressure of normotensives.

In still another embodiment of the present invention, the use is for treating vasoconstriction.

In still another embodiment of the present invention, the use is for treating superoxide and nitric oxide-induced free radical oxidative damage.

In still another embodiment of the present invention, the use augments the level of oxygen scavenging enzymes comprising superoxide dismutase (SOD), and catalase.

In still another embodiment of the present invention, the use decreases the level of thiobarbituric acid reactive substances (TBARS).

In still another embodiment of the present invention, the use is for treating various kinds of edema selected from brain and pulmonary edema.

A method of obtaining a high yield lipid soluble extract and its subsequent fractions comprising a fraction A consisting of ar-turmerone of formula 1, and turmerone of formula 2, a fraction B consisting of curcumene and zingiberine, and fraction C consisting of germacrone, curcumerone, zedoarone, sedoarondiol, isozdedoaronidiol, curcumenone, and curlone, from rhizomes and leaves of *Curcuma species* of the Zingiberaceae family, comprises the steps of:
- powdering the rhizomes and leaves of the curcuma species in fine particle form,
- extracting the said powder with a polar organic solvent under continuous stirring or sonication for about 24 hours at room temperature,
- repeating the extracting step two to five times,
- removing the solvent by distillation under reduced pressure and below about 45°C to obtain a residual concentrate,
- triturating the residual concentrate with a non-polar solvent,
- removing the solvent by distillation under reduced pressure and below 45°C,
- obtaining the said lipid soluble extract,
- fractionating the said extract by column chromatography,
- obtaining fraction A, fraction B, and fraction C, and
- fractionating each of fractions A, B, and C further using HPLC or GLC to obtain the said constituents.

An embodiment of the method includes fractioning the extract on silica gel column.

In another embodiment of the method, the polar solvent is selected from alcohol and acetone.

In still another embodiment of the method, hte non-polar solvent is selected from light petroleum and toluene.

Another embodiment of the method includes fractionating the extract using n-hexane, n-hexane: ethyl acetate mixture of ratio 95: 5, and ethyl acetate successively.

In still another embodiment of the method, fraction A constitutes about 75% of the said extract.

In still another embodiment of the method, ar-turmerone constitutes 95% of the fraction A.

In still another embodiment of the method, the pressure ranges between 7 and 11 mmHg.

In still another embodiment of the method, the concentration of the extract is about 6%.

Another embodiment of the method includes powdering dry rhizomes and leaves into fine particles.

Another embodiment of the method includes percolating said powder with organic solvent at room temperature.

Another embodiment of the method includes stirring the contents continuously during percolation.

Another embodiment of the method includes removing the said organic solvent by distillation under reduced pressure below 45°C.

In still another embodiment of the method, the above mentioned percolation step is repeated at least 4-8 times.

Another embodiment of the method includes collecting *Curcuma* oil as orange yellow odoriferous liquid at 5-8% yield.

Another embodiment of the method includes separating said oil into its constituents by using techniques like Chromatography and distillation under high vacuum.

In still another embodiment of the method, *Curcuma* species is selected from *Curcuma longa* L. Syn. *Curcuma domestica* Valeton, and *Curcuma aromatica* Salisb.

In still another embodiment of the method, the organic solvent is a non-polar organic solvent selected from light petroleum, and toluene.

In still another embodiment of the method, the organic solvent is a polar organic solvent selected from ethanol, and propanol.

In still another embodiment of the method, non-polar organic solvents give higher yields as compared to polar organic solvents.

In still another embodiment of the method, the residual concentrate from the polar organic solvent extract is extracted with non-polar organic solvent.

In still another embodiment of the method, *Curcuma* oil is separated into its individual constituents comprising ar-d-turmerone (formula 1), turmerones of α and β (formula 2), zingiberine, curcumene, germacrone, curcumenone, and curlone.

In still another embodiment of the method, the kind of the Chromatography is selected from Column Chromatography, preferably High Performance Liquid Chromatography, and Gas-Liquid Chromatography.

In still another embodiment of the method, the adsorbent for the Chromatography is selected from alumina, and silica gel.

In still another embodiment of the method, the elution of the said constituents is with organic solvent selected from n-hexane, ethyl acetate, and n-hexane and ethyl acetate mixture in varying proportions.

In still another embodiment of the method, molecular weight of the individual constituents of *Curcuma* oil separated by chromatography is turmerones (α-, β-) - mol.wt. 218, ar-d-turmerone - mol.wt. 216, zingiberine - mol.wt. 204, and Curcumene - mol.wt. 202.

In still another embodiment of the method, retention time of the individual constituents of *Curcuma* oil separated by chromatography is turmerones (α-, β-) - retention time 9'-04", ar-d-turmerone - retention time 8'-08", zingiberine - retention time 5'-04", and Curcumene - retention time 4'-24".

The pharmaceutical composition is for treatment of neurocerebrovascular disorders, said composition comprising an effective amount of the lipid soluble extract called *Curcuma* oil, from rhizomes and/or leaves of species of plant Zingiberaceae family particularly *Curcuma* species, either as such or its individual major active constituents in combination with each other, optionally associated with pharmaceutically acceptable additives or related compounds comprising melatonin, and tissue plasminogen activator (t-PA).

In still another embodiment of the present invention, the composition is for use to treat, reduce, control and prevent diseases conditions relating to increased production of nitric oxide (NO), injury due to inflammation, increased calcium entry and free radical oxidative damage to important biomolecules.

In still another embodiment of the present invention, the additive is selected from nutrients comprising proteins, carbohydrates, sugar, talc, magnesium stearate, cellulose, calcium carbonate, starch-gelatin paste, and/or a pharmaceutically acceptable carrier, excipient, diluent, or solvent.

In still another embodiment of the present invention is for administration orally, inhaled, or implanted.

In still another embodiment of the present invention, the physical state of the said composition for the oral route is in the form of capsule, tablet, syrup, concentrate, powder, granule, aerosol, or beads.

In still another embodiment of the present invention, the composition is for administration at a dosage level ranging between 5 to 5000 mg/day.

In still another embodiment of the present invention, it is for treating animals or human beings.

In still another embodiment of the present invention, it is for treating hypertension.

In still another embodiment of the present invention, it is for treating cerebral and pulmonary edema which accompanies cerebral and myocardial infarction.

In still another embodiment of the present invention, it is for treating reperfusion injury.

In still another embodiment of the present invention, it is for treating cerebrovascular diseases comprising strokes, and transient ischaemic attacks.

In still another embodiment of the present invention, it is for treating all kind of strokes comprising thromotic, embolic, focal and recurrent.

In still another embodiment of the present invention, it is for treating subarachnoid and cerebral hemorrhage.

In still another embodiment of the present invention, it is for treating neurological dysfunction.

In still another embodiment of the present invention, it is for treating thrombosis infarction comprising cerebral, coronary, and deep vein.

In still another embodiment of the present invention, it is for treating cancer.

In still another embodiment of the present invention, it is for treating Alzheimer's disease wounds.

In still another embodiment of the present invention, it is for treating Acquired Immunodeficiency Syndrome.

In still another embodiment of the present invention, it is for treating migraine.

In still another embodiment of the present invention, it may be administered again in case of relapse conditions.

In still another embodiment of the present invention, the composition is for treating post- stroke injury.

In an embodiment of the present invention, therapeutically effective medicaments are obtained or prepared from extracts of *Curcuma* species rhizomes and leaves which belong to the Zingiberaceae family.

In another embodiment of the present invention, more particularly, the lipid soluble extract/fraction is from *Curcuma longa* L. syn., *Curcuma domestica* Valeton, commonly know as *turmeric* or *Haldi.*

In another embodiment of the present invention, pharmaceutically acceptable formulations /delivery systems such as tablets, capsules, suppository, beads, aerosols, etc. may be used for the treatment and prevention of human diseases in which increased production of Nitric Oxide (NO) and free radical oxidative damage are implicate.

Such diseases are neurocerebrovascular disorders like transient ischaemic attacks (ischaemic, hemorrhagic, focal recurrent etc.) thrombosis (cerebral, coronary, deep vein), infarction, stroke (thrombotic, embolic, focal etc.), Alzheimer's disease, inflammatory and neurological dysfunctions, wounds, carcinogenesis, tumor progression etc.

The superoxide and nitric oxide (NO) scavenging property of the lipid soluble extract/fraction of *Curcuma* species rhizomes (Family: Zingiberaceae) especially *Curcuma longa* L. Syn. *Curcuma domestica* Valeton, hereinafter referred to as *Curcuma* oil either as such or its major active constituents in combination with each other makes them therapeutically effective to control various degenerative diseases, more particularly as a drug which is a nitric oxide (NO) and superoxide scavenger with anti-inflammatory activity to combat brain and pulmonary edema/swelling which accompanies brain and myocardial infarction.

Keeping these biological profiles in view and as a follow-up of the holistic view of Ayurveda of human diseases, the lipid soluble extract/ material of *Curcuma* species rhizomes and leaves (*Zingiberaceae* family) hereinafter referred to as *Curcuma* oil and obtained from *Curcuma longa* L. syn. *Curcuma domestica* Valeton, rhizomes and leaves, either as such or its major active constituents, ar-d-turmerone (formula 1), turmerones (α-, β-, formula 2) in combination with each other with and without the other minor constituents are found to be significantly beneficial and possess powerful nitric oxide (NO) and free radical/superoxide scavenging activity.

Said lipid soluble extracts exhibit/possess potent free radical scavenging/antioxidant activity which enables them to protect mitochondrial impairment protecting downstream target and they inhibit overproduction of nitric oxide synthase (NOS), avoid injury due to inflammation and reduce calcium entry so that the resultant calcium overload in the neurons does not occur.

Another important advantage is that if there is any blockage, the above three parameters which are the major cause of reperfusion injury are taken care of by these medicaments and the collaterals from the "Circle of Willis" are able to help in the blood flow and thereby enable the drug to reach the site of injury.

In cases where severe brain ischaemia has occurred, administration of *Curcuma* oil either as such or its individual major active constituents ar-d-turmerone and turmerones in combination with each other with and without other therapeutically beneficial agents such as melatonin, other antioxidants, calcium channel antagonists, tissue plasminogen activator (t-PA) and cell membrane stabilizing agents can provide effective protection against cerebral and even coronary damage.

Since stroke is one of the main causes of the mortality among hypertensive patients, our findings also underline the importance of the *Curcuma* oil either as such or its individual major active constituents in combination with each other as an effective antihypertensive drug with antioxidant and neuro protective activities.

The lipid soluble extract of rhizomes and leaves of *Curcuma* species of the family zingiberaceae especially *Curcuma longa* L. syn. *Curcuma domestica* Valeton hereinbefore referred to as *Curcuma* oil which is a pale yellow to orange yellow odoriferous oily liquid whose major constituents are: ar-d-turmerone (formula 1), turmerones (α-, β-, formula 2) [Tap Chi Hoa Hoc:25, 18 (1987); Chem. Abstr.,108, 137682s (1988)] besides other minor constituents such as zingiberine, curcumene, curlone, curculone, curzenone, α-, β- curcumenolides, curcumenone, curdione, germacrone, linalool, camphor, borneol, zingiberol etc. [Essenze Deriv. Agrum, 54, 117 (1984); Chem Abstr.,103,128791w(1985)] inhibits increased production of nitric oxide (NO) and is a free radical scavenged antioxidant which can penetrate the blood brain barrier and provide effective therapeutic protection by combating nitric oxide (NO) and superoxide/free radical induced neuronal injury /damage in human diseases such as neurocerebrovascular dysfunctions, all types of strokes, thrombosis (cerebral, coronary, deep vein), infarction, inflammatory and neurological disorders, certain types of cancer, wounds, Alzheimers disease and other nitric oxide neurotoxicity,hyperbaric oxygen exposure etc.

High yields. of the lipid soluble material can be obtained from *Curcuma* species rhizomes and leaves (Family: Zingiberaceae), particularly *Curcuma longa* L. syn. *Curcuma domestica* Valeton, hereinafter referred to as *Curcuma* oil and isolation of its various constituents.

More particularly this invention relates to nitric oxide (NO) and superoxide scavenging activity and prevention of any changes in cerebral blood flow dynamics by *Curcuma* oil itself or by its major active constituents in combination with one another which enables their use as medicaments for the treatment and prevention of neurocerebrovascular disorders and related and unrelated dysfunctions such as ischemic attacks, all types of stroke, thrombosis, infarction, migraine, Alzheimer's disease, inflammatory and neurological dysfunctions, carcinogensis, tumor progression wounds and even HIV/AIDS.

### Brief description of the accompanying drawings

Fig. 1 shows prevention of infarction from focal ischaemic rat after using curcuma oil;
Fig. 2 shows past occlusion complete prevention of infarction in forebrain after using fraction A;
Fig. 3 shows past occlusion complete prevention of infarction in forebrain after using fraction B ;
Fig. 4 shows Calcium transients in mitochondria (340/380 ratio);
Fig.5 shows change in SOD levels in mitochondria after using fraction A and fraction B;
Fig. 6 shows Catalase levels in mitochondria after using fraction A and fraction B;
Fig. 7 shows Malondialdehyde levels in mitochondria after using fraction A;
Fig. 8 shows change in percent relaxation to NE induced contraction ; and
Fig. 9 shows change in SNP levels in mitochondria after using fraction A.

A method of obtaining the lipid solubleextract/fraction of rhizomes and leaves of various species belonging to zingiberaceae family, especially *Curcuma* oil from *Curcuma* species in good yield is by extraction of powdered rhizomes/leaves of *Curcuma longa* L. Syn. *Curcuma domestica* Valeton or *Curcuma aromatica* Salisb or *Curcuma zedoaria* Roxb. etc. with an organic solvent like alcohol, acetone, ethyl acetate etc. but preferably a non-polar organic solvent like light- petroleum or toluene under constant stirring and removing the solvent from such extracts by distillation under reduced pressure below 45°C.

In the case of extraction by a polar organic solvent such as ethanol, the residual alcoholic concentrate after removal of the solvent is exhaustively extracted with a non-polar organic solvent like light-petroleum, toluene etc.

Distillation of solvent from such extracts under reduced pressure below 45°C yields a pale yellow to orange yellow odoriferous liquid in 5 to 6 per cent yields.

Fractionation of this oil by column chromatography over a suitable adsorbent and elution by an appropriate organic solvent or by HPLC or GLC or distillation in vacuum yields the individual constituents such as ar-d- turmerone (formula 1), turmerones (α-, β-, formula 2) as major constituents (about 70 percent as determined by GC-MS) besides other minor constituents like zingiberine, curcumene, zedeorone, germacrone, curlone, curdione etc. all identified by GC-MS etc.

An improved method of obtaining the lipid soluble extract/material of rhizomes and leaves of various species of Zingiberaceae family especially *Curcuma* species such as *Curcuma longa* L. Syn. *Curcuma domestica* Valeton or *Curcuma aromatic* Salisb. etc. in high yields is by exhaustive extraction of finely powdered rhizomes or leaves with an appropriate organic solvent under continuous gradual stirring or by sonication at ordinary room temperature followed by removal of the solvent from the extract by distillation under reduced pressure below 45°C.

In an embodiment of the method, the organic solvent is a non-polar organic solvent such as light petroleum, toluene etc.

In another embodiment of the method, the organic solvent used is a polar organic solvent such as ethanol, propanol etc.

In another embodiment of the method, the residual concentrate after removal of the solvent from the polar organic solvent extract is exhaustively extracted with a non-polar organic solvent such as light-petroleum, toluene etc.

In another embodiment of the method, the organic solvent is removed from the extracts by distillation under reduced pressure below 45°C.

In another embodiment of the method, the continuous stirring is done either manually or by a mechanical stirrer or by an electric motor.

The lipid solubleextract/material of rhizomes or leaves of said species, which, is a pale yellow to orange-yellow odoriferous oily liquid, can be separated into its individual constituents such as ar-d-turmerone (formula 1), turmerones (α-, β-, formula 2), zingiberine, curcumene, germacrone, curcumenone, curlone etc. by chromatography (column, HPLC, GLC etc. ) or distillation under high vacuum.

The individual constituents of the *Curcuma* oil can be obtained by column chromatography over a suitable adsorbent such as alumina, silica gel etc. and elution by appropriate organic solvents such as n-hexane, n-hexane : ethyl acetate mixture (in varying proportions), ethyl acetate etc.

The individual constituents *of Curcuma* oil can be obtained by HPLC or GLC, e. g. turmerones (α-, β-), mol.wt. 218, retention time 9'-04", ar-d-turmerone (mol.wt. 216), retention time 8'-08", zingiberine (mol.wt. 204) retention time 5'-04", Curcumene (mol.wt. 202), retention time 4'-24".

The individual constituents of *Curcuma* oil, such as ar-d-turmerone (formula 1), turmerones (formula 2), zinziberine, curcumene, curcumenone, germacrone etc. are obtained by distillation of *Curcuma* oil in vacuum, e. g. ar- d-turmerone, b.p. 155-160°C / 9mm Hg through ar-d-turmerone rich fraction, b.p. 140-160°C / 10mm Hg which is about 70 % of the whole *Curcuma* oil.

Therapeutically beneficial effects of *Curcuma* oil as such or its individual major active constituents in combination with each other reduce, control or prevent human diseases in which increased production of nitric oxide (NO) and free radical oxidative damage to important biomolecules is implicated such as all types of stroke (thrombotic, embolic, focal, ischaemic), thrombosis (cerebral, coronary, deep vein) infarction, neurological dysfunctions etc.

In another embodiment of the present invention, use for treating post-stroke injury in mammals is by administration to a subject in need thereof of an effective amount of *Curcuma* oil either as such or its individual major active constituents in combination with each other.

In another embodiment of the present invention, use for treating patients of subarachnoid and cerebral hemorrhagic stroke after 5 to 7 hours of the stroke is by administration to a subject in need thereof of a therapeutically effective amount of *Curcuma* oil either as such (whole) or its individual major active constituents in combination with each other.

In another embodiment of the present invention, use for treating reperfusion injury in mammals is by administration to a subject in need thereof of an effective amount of *Curcuma* oil (whole-as such) or its individual major active constituents in combinations with each other.

In another embodiment of the present invention, use for treating cerebrovascular diseases like all types of stroke (thrombotic, embolic, focal, recurrent), transient ischaemic attacks etc. is by administration to a subject in need thereof of an effective amount of *Curcuma* oil (whole-as such) or its individual major active constituents in combination with each other.

In another embodiment of the present invention, use for treating ischaemic diseases and preventing dangerous blood clot formation is by administering to a subject in need thereof of an effective amount of *Curcuma* oil (whole) or its individual major active constituents in combination with each other.

In another embodiment of the present invention, use for treating hypertension in mammals is by administration to a subject/patient in need thereof of an effective amount of *Curcuma* oil (whole) or its individual major active constituents in combination with each other.

In another embodiment of the present invention, use to combat cerebral and pulmonary edema which accompanies cerebral and myocardial infarction is by administration to a subject in need thereof of medicaments comprising *Curcuma* oil (whole) or its individual major active constituents in combination with each other.

The lipid soluble extracts, either as such or its individual constituents like ar-d-turmerone, turmerones, germacrone, zinziberine, curcumene, curlone etc. in combination with each other with and without other therapeutically useful agents such as melatonin, tissue plasminogen activator (t-PA) administered orally, parentally (individual pure constituents) or in any other appropriate pharmaceutically acceptable delivery system such as tablets, capsules, beads, suppositories aerosols, implants etc in an effective amount (for stroke, 10-500 mg/daily in divided doses and for other ailments, 10-1000 mg/daily in divided doses), provide a highly effective cure/treatment for human diseases wherein nitric oxide (NO) and free radical oxidative damage are implicated such as all type of stroke, thrombosis, infarction and neurological dysfunctions and may also be of therapeutic use in certain type of cancer such as leukemia, Alzheimer's disease wounds and even HIV / AIDS.

### Examples

### Example 1

This example describes the method of obtaining *Curcuma* oil and its constituents in high yields and preparation of its dosage formulations, Improved extraction procedure of *Curcuma* oil and its constituents from *Curcuma longs* L. syn. *Curcuma domestica* Valeton or other *Curcuma* species rhizomes.

The usual extractive procedure employs three or four percolations of dry powdered *Curcuma* rhizomes with an organic solvent like light Petroleum, toluene, alcohol etc. and distillation of the solvent from the percolates. In case of alcoholic extracts, after solvent removal the residual concentrate is triturated with a non-polar organic solvent like light petroleum followed by removal of the solvent by distillation to yield *Curcuma* oil in 1 to 1.5 percent yields.

Hot extraction (Soxhlet) leads to loss of essential volatile constituents. When these procedures were changed to extraction of the dry powdered *Curcuma* rhizomes with appropriate organic solvents such as light petroleum, acetone, alcohol etc. with continuous stirring by mechanical stirrers driven by electric motors or manually or agitation with sonicator followed by remove of the solvent from the extracts by distillation under reduced pressure below 45° C the yield and quality of the *Curcuma* oil increased appreciably.

In a typical procedure, dry finely powdered *Curcuma longa* L. rhizomes (1 kg) were successively percolated with n-hexane (3 liters) in a stainless steel or glass percolator / vessel fitted with a tap near the bottom to drain out the percolate, and the contents were stirred under slow motion continuously for 24 hours each time by a mechanical stirrer driven by an electric motor. The orange-yellow percolate was drained out and the procedure repeated four to five times. Solvent was distilled off from the percolates under reduced pressure below 45 °C to yield an orange yellow odoriferous liquid (51 gms =5.1% yield).

Likewise, initial extraction of finely powdered *Curcuma longa* L rhizomes (1 kg) with acetone or alcohol (5x 3 liters) under continuous stirring for 24 hours each time followed by removal of the solvent from the percolates by distillation under reduced pressure below 45°C and exhaustive trituration of the residual concentrate with n-hexane or toluene (6x500 ml) followed by removal of the solvent by distillation under reduced pressure below 45°C yielded an orange yellow odoriferous liquid (60 gm.=6% yield).

Column chromatography of this orange yellow odoriferous liquid (20.0 gm.) over a silica gel column, using n-hexane, n-hexane: ethyl acetate (in varying proportions) mixture and ethyl acetate successively gave ar-d-turmerone (formula 1, 55%) and turmerones (α-,β- formula 2, 20%) as major constituents (fraction- A.) followed by curcumene(10%) & zingiberine (fraction- B) and other minor constituents -germacrone, curcumerone, zedoarone, zedoarondiol, isozdedoarondiol, curcumenone, curlone etc. (fraction C) whose activity was low..

Distillation of *Curcuma* oil (20.0 gms.) under reduced pressure (140-160°C / 9mm Hg) yielded ar-d-turmerone rich major fraction I (formula 1, 15.0 gms) alongwith other turmerones (α-,β-, formula 2) and other minor constituents (4.2 gms, fraction II) Fraction I 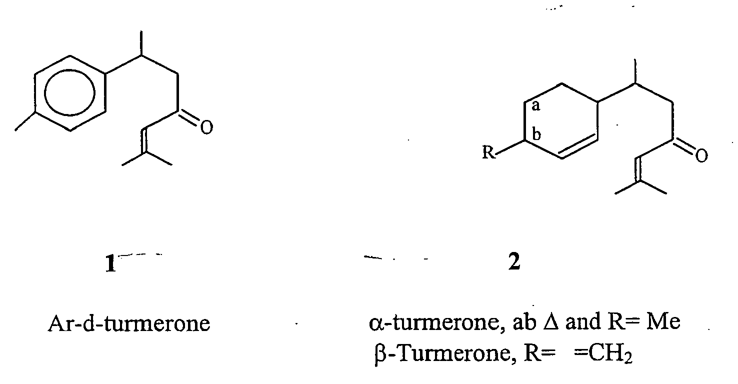 had refractive index (n_{D}³⁰) 1.4990, specific optical rotation (L)²⁵+19.6. *Curcuma* oil itself or its individual constituents obtained by chromatography or distillation under high vacuum are used singly or in combination with each other with and without other therapeutically beneficial compounds to prepare appropriate clinically effective formulations.

The solid dosage form may be obtained by maceration of *Curcuma* oil as such or its individual constituents singly or in combination with each other particularly ar-d-turmerone, α-,β- turmerones with starch and microcrystalline cellulose in suitable proportions in a mixer till the mixture becomes a free flowable powder which may be filled in capsules or converted into tablets as per therapeutically desired specifications.

In a typical example, *Curcuma* oil (10.0 gm.) was dissolved in ethanol (100ml). Starch (5.0gm) and microcrystalline cellulose (85.0 gm) were added to this solution. The contents were mixed thoroughly and solvent was removed by drying below 45°C. The resulting product was passed through 40-mesh size sieve to obtain free flowing granules. These granules were then compressed into tablets of appropriate dosage requirements, e.g. each tablet weighing 500mg.contain 50mg of *Curcuma* oil.

### EXAMPLE 2

### Focal cerebral ischaemia:

Male Sprague Dawley rats of 270-375 gm weight from CDRI Animal House were used for this study. Rats were housed in a 12-hr. light / dark cycle and water was given ad libitum. Animals were fasted overnight and anaesthetized with pentobarbitone sodium, 30mg/kg. Rectal temperature was monitored. Transient ischaemia / reperfusion was performed using an intravascular filament to occlude the middle cerebral artery unilaterally [Longa Z.E.,Weinstein P.R., Carlson S., Cummins R.; Reversible middle cerebral artery occlusion without craniectomy in rats : Stroke ,20, 84-91(1989) ] for 2 hours followed by reperfusion for the remainder of 36 hours. Animals were assigned randomly to the following groups of n=5 rats (1) Control: Sham operated. (2.) Ischaemic / reflow- no treatment. (3.) Ischaemic /reflow-treated group:( i) *Curcuma* oil (weight/ml., 0. 86gm.), 683.65 mg./kg., given i.p. and P.O. ( ii) . Fraction- A(weight /ml.,0. 88gm.), 569. 56 mg/kg., given , i.p. and P.O. ( iii ). Fraction -B. ( weight /ml.,0. 91gm.) 938.86mg / kg., given, i.p. and P.O. The animals were sacrificed & brains were removed and quickly frozen. Eight coronal section of of 2mm thickness from each brain were cut and stained with 2,3,5-triphenyltetrazolium chlorine at 37° C for 30min. and post fixed by formalin. Each brain slice was photographed. The area of infarct in each slice was evaluated in a double blind manner. From groups (1,2,&3) rats n=3 , brain was removed and processed for mitochondrial Ca²⁺ estimation.

### Experimental Protocol:

### Isolation of forebrain mitochondria:

Mitochondria were isolated from the rat forebrain according to the method of Lai and Clark [Lai. J.C.K., Clark J.P., Preparation of synaptic and non-synaptic mitochondria from mammalian brain: Method Enzymol., 55, 51-60(1979)] with slight modifications. Rat forebrain was immediately removed after decapitation and immersed in ice- cold isolation medium or Phosphate Buffered Saline. Brains were minced and rinsed to remove all the traces of blood. The tissue was homogenized (10% w/v) in an appropriate medium using a motorized Teflon homogenizer and immediately centrifuged at 1800g for 10 min. The supernatant was decanted and the pellet rehomogenized and centrifuged at 1800 g for 10 min. Supernatants from the first and the second spins were added together and centrifuged at 17,000 g for 20 minutes. The resultant pellet was resuspended in specific mediums and centrifuged at 17000 g. for 5 minutes.

### Determination of mitochondrial content:

Calcium content of mitochondria isolated from forebrain was estimated according to the method of Zaidan E. and Sims N.R. [The calcium content of mitochondria from brain sub regions following short term fore brain ischaemia and recirculation in the rat; J. Neurochem., 63,1812-1819(1994)], with slight modifications . In brief , mitochondria (0. 3 mg. protein) in succinate mediun were loaded with Fura-2AM (0-5µM) and incubated for 30 min. at 37°C with constant shaking. The mitochondria were then washed twice in succinate medium and re-suspended in the same medium.

The ratio of Fura-2 fluorescence at exciting wavelength of 340 and 380 nm with emission at 510 nm was determined using a Shimadzu RF 5000 Spectrofluorometer. Mitochondrial Calcium([Ca ²⁺]m), is presented as tracings of the 340/380 fluorescence ratio[Macleod K.T and Harding S.E.; Effect of phorbolester in contraction, intracellular pH and intracellular Ca2+ in isolated mammalian ventricular myocytes. J. Physiol. (London) ,444, 481-498 (1991)].

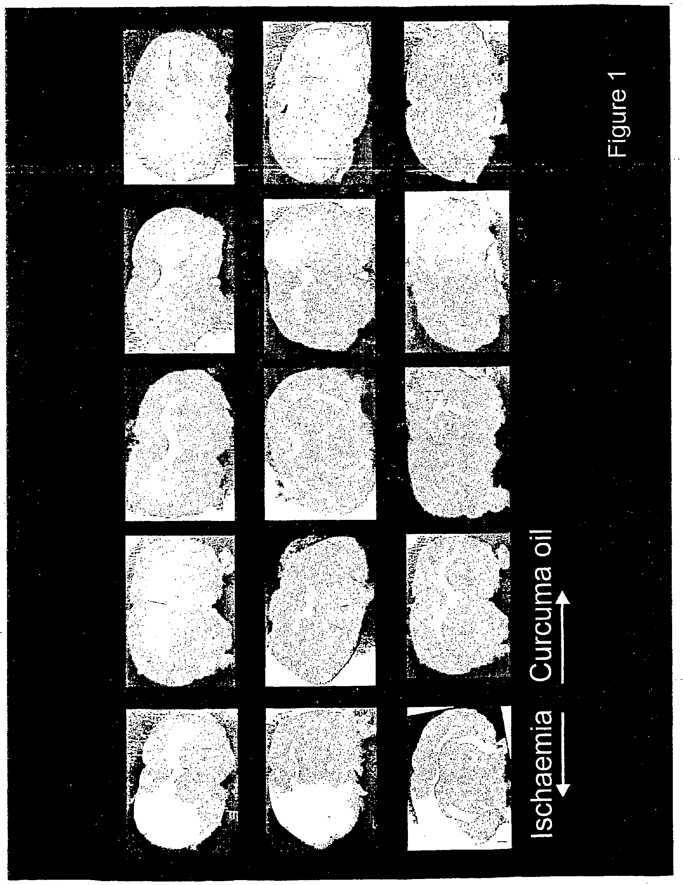

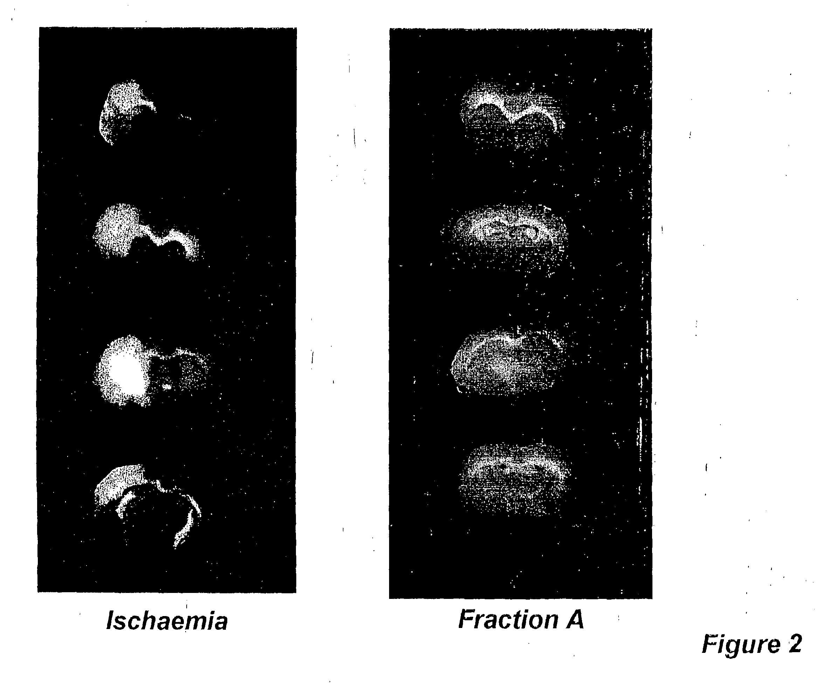

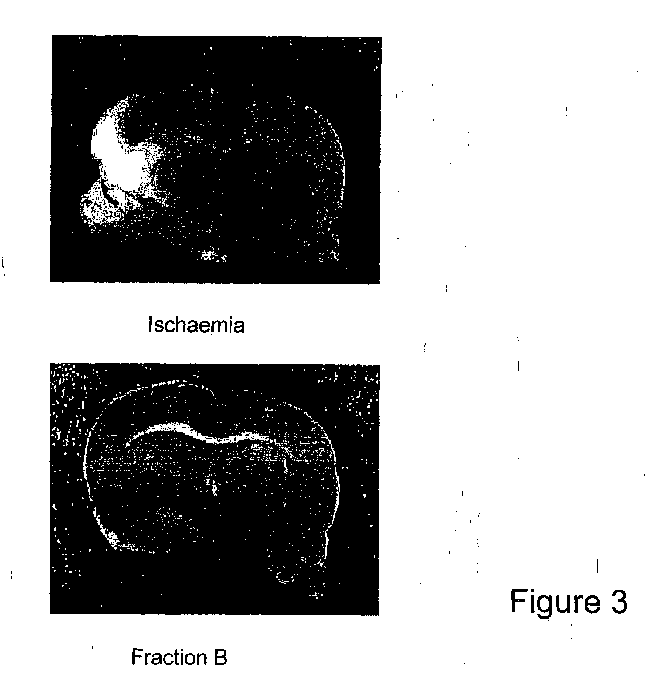

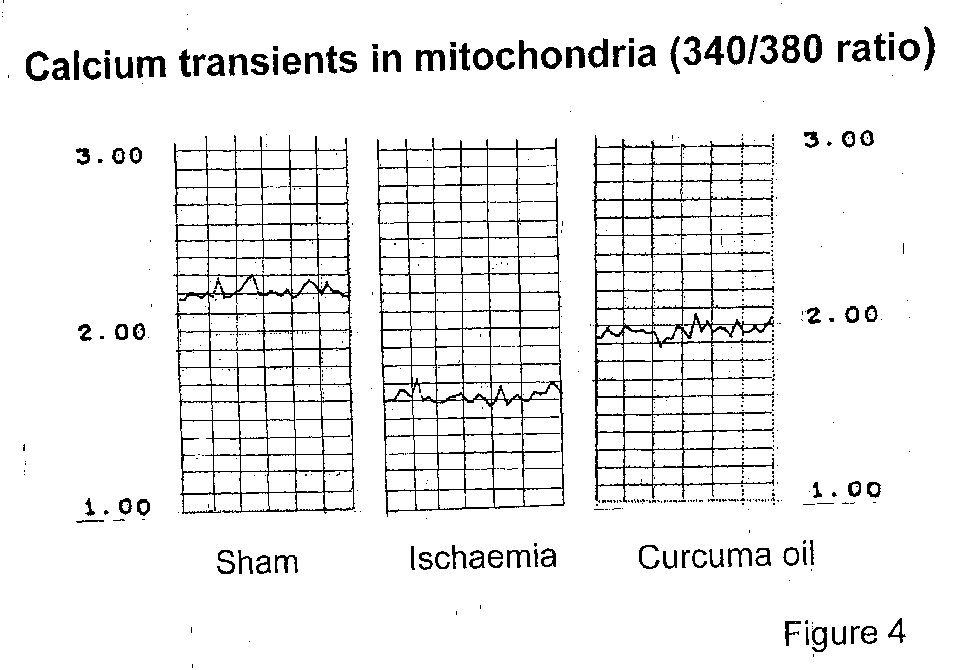

Result : Infarct from focal ischaemic rat in pretreated group was completely prevented as seen in Fig. 1 & 2. In the group where test compound / agent was given post occlusion of middle cerebral artery, six out of seven brain sections shows complete prevention (Fig.3), whereas in one about 20% of the area showed up as infarcted. Mitochondria isolated from forebrain from animals made sham, ischaemc and treated with the test compound group (i) showed the intracellular calcium levels close to normal (Fig.4).

### Example 3

### Collagenase-induced intra-cerebral hemorrhage:

Adult male rats (250-350 gm.) from the CDRI-Animal House were used in the following experiments. The rats were anaesthetized with pentobarbitone sodium (30 mg/kg, i.p.) and placed in a stereotaxic frame (for rats, Narashige, Japan). Rosenberg et. al's method [Rosenberg G.A. Mun-Bryce S.,- Mary B.S. and Kornfeld M., Collagenase-Induced intracerebral hemorrhage in rats: Stroke, 21, 801-807 (1990)] was followed. An incision was made in the scalp and a 23- guage needle was implanted into the caudate nucleus and the putamen (at the coordinates of A5.8, L3.0, H1). ( A stereotaxic atlas of the rat brain, eds.R. M. Elliot, Gardener Lindzey and Kenneth, MacCorquodale. Meredith Publishing Company, 1967). Rats(n=10) were injected with collagenase (0.01 IU in 2µl of saline) and for sham with 2 µl of normal saline. After infusion, the needle was removed and the wound was sutured. The animals were allowed to recover from anesthesia, kept in a warm place and allowed access to food and water. Eighteen hours later, the animals were evaluated for neurological deficit by measuring scoring of abnormal possture and hemiplegia according to Yamamoto et.al. [ Yamamoto A.Tamura, Kirino T., Shimizu M and Sano K. Behavioral changes after focal cerebral ischaemia by left middle cerebral artery occlusion in rats. Brain Research ,452, 323-328.(1988)]. Later rats were reanaesthetized with pentobarbitone sodium, 30-mg/kg i.p. and brain was removed. Rats were assigned randomly into three groups. Group 1, received saline. Collagenase (0.01 IU in 2µl saline) treated Groups 2 & 3 received fraction A (683. 65mg/kg) after 5 and 7 hours of collagenase treatment by the oral route.

### Antioxidant estimations

Mitochondria were isolated as described in example 2.For antioxidant estimations, the mitochondria were rinsed and suspended in phosphate buffer. For the other estimations mitochondria were resuspended in a medium containing sucrose 250 mM, KH₂PO₄ 6 mM and succinate 6mM, pH 7.2. The isolation procedure was carried out at 4°C,

### Antioxidants:

The oxygen scavenging enzymes, superoxide dismutase (SOD), catalase (CAT) and thiobarbituric acid reactive substances were estimated in mitochondria isolated from forebrain of experimental animals.

**SOD:** SOD activity was measured by the inhibition of NADH, PMS, NBT and absorbance monitored at 560 nm. Enzyme activity is expressed in U/min/mg protein.[Nishikini M., Rao N.A., Yagi K., The occurrence of superoxide anion in the reaction of reduced PMS and molecular oxygen. Biochem Biophysi. Res. Commun., 46, 849- 854 (1972)].

**CAT:** CAT activity was assayed by measuring the UV absorbance change of H₂O₂ at 240 nm according to Aebi [Aebi H., In Methods of Enzymatic Analysis (Third edition) ed. H.U. Bergmeyer Academic Press, New York and London, Vol.2 pp 673-684. (1974)]

### Thiobarbituric acid reactive substance (TBARS):

Mitochondrial TBARS levels were measured as an index of malondialdehyde and hence lipid peroxidation by the method of Okhawa et. el.[Okhawa H., Ohishi N., Yagi K., Assay of lipid peroxides in animal tissues by thiobarbituric acid reaction: Anal. Biochem., 95, 351 (1979)] at 532 nm. Functional deficit was estimated according to Bederson [Bederson J.B., Pitts, L.H. Tsiji M., Nishimura M.C. Davis R.L., Barkowisk H., Rat MCAO: Evaluation of model and development of a neurologic examination, Stroke, 17, 472 (1986)] and water contents were estimated. Both the parameters were found to be significantly reduced as compared to untreated group.

### Protein Assay

Mitochondrial protein was determined by the method of Lowry et.al. [Lowry O.H., Rosbrough N.J., Farr. A.L., Randall K.J.; Protein measurement with folin phenol reagent: J. Biol. Chem., 193, 265 (1951)] using bovine serum albumin(BSA) as standard.

Result: The test compound (fraction A) given 5 hours after collagenase treatment significantly reduced the edema. Neurological deficit at 5 & 7 hours of treatment were scored as grade 4 in untreated group and grade 0-2 in treated group. Mortality in untreated group was 3 out of 5 and in treated group 1 out of 5.

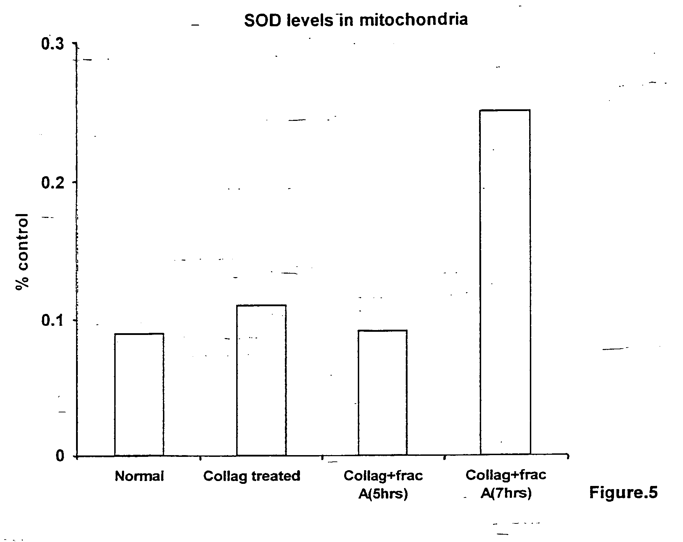

**SOD:** SOD value in 5 hours was almost normal while in case of test compound (fraction A) given after 7 hours post collagenase treatment the SOD levels were augmented (Fig. 5).

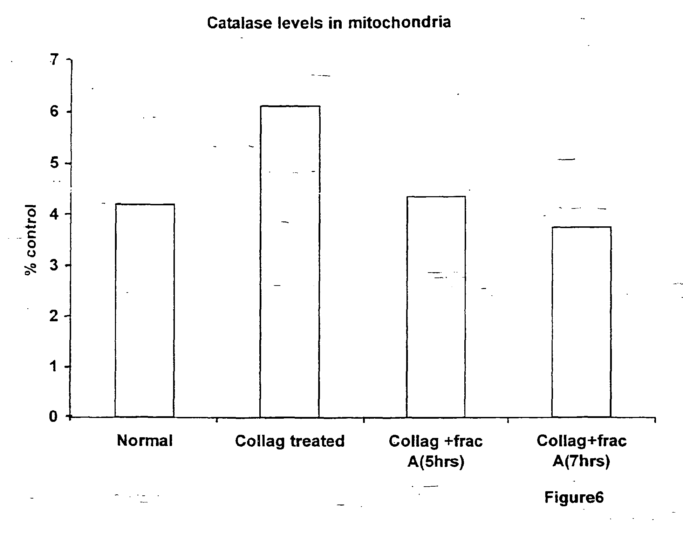

### Catalase: This enzyme is reported to be present in minute amount in brain (Fig. 6)

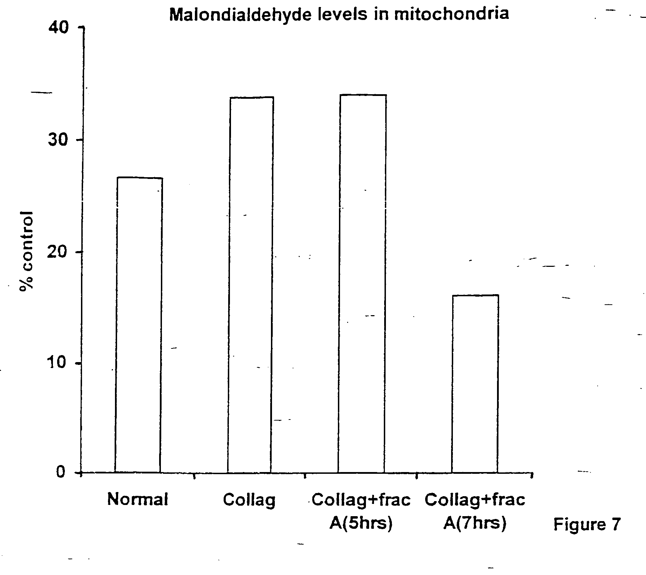

**TBARS:** At 5 hours post collagenase treatment, the values were close to that of collagenase treated animals, while at 7 hours the values were decreased significantly as compared to the normal group indicating the anti-oxidant property of the test compound-fraction A (Fig.7).
Mitochondria were isolated as described in Example 2.

### Example 4

Adult male rats (250-350gm.) from the C.D.R.I. Animal house were anaesthetized with 30mg./ kg. Pentobarbitone sodium. Jugular veins of the rats were exposed. Five drops of 10 % formalin in 65 % methanol was dropped on the vein. Six hours were allowed for thrombus formation which was then graded according to its presence or absence. [Blake O. R., Ashwin J.G., Jaques L.B.,; An assay for the antithrombotic activityofanticoagulants:J.Clin.Pathol.,12,118 (1959)]. Fraction A (ar-d-turmerone and turmerones) was given 200µl.i.p./300gm.rat in the treated group,while the untreated (control) group received equivalent amount of saline (i.p).
Result: The thrombus in the untreated group was 2.8mg. and in the treated group it was 0.75mg. showing an increase of 373.33 % in untreated versus treated group.

### Example 5

Rats were made hypertensive according to Goldblatt et. al.[ Goldblatt H., Lynch J., Hanezal R.F., Serville Mi.W.: Studies on experimental hypertension,: The production of persistent elevation of systolic blood pressure by means of renal ischaemia J Exp Med ;59:347-379 (1934)]. Eight weeks later the hypertensive rats had an average initial blood pressure of 200 mm/Hg. After *Curcuma* oil , 683.65mg./kg. was administered intraperitoneally the blood pressure fell to 115 mm /Hg in 15 min. and stayed at that level for more than 60 min.

**Table 1**

| | Dose (i.p.) | Blood pressure | Duration | No.of Expt. |
|---|---|---|---|---|
| Rats | (683.65mg/kg). | Fall(%) | (min.) | |
| Hypertensive | | 38.76± 7.19 | >60 min. | n = 3 |
| Normotensive | | No fall | | n = 2 |

Result: The compound lowers the blood pressure significantly in hypertensive rats and not in the normotensive rats. It reduces blood pressure without bradycardia due to β- adrenergic receptor antagonism or reflex tachycardia common to vasodilator [Nichols A.J,Gallai M.Ruffolo R.P Jr. Studies on the mechanism of arterial vasodilation produced by the noval antihypertensive agent. Carvediolol. Fundam. Clin. Pharmacol., 5:25-38 (1991) ].

### Example 6

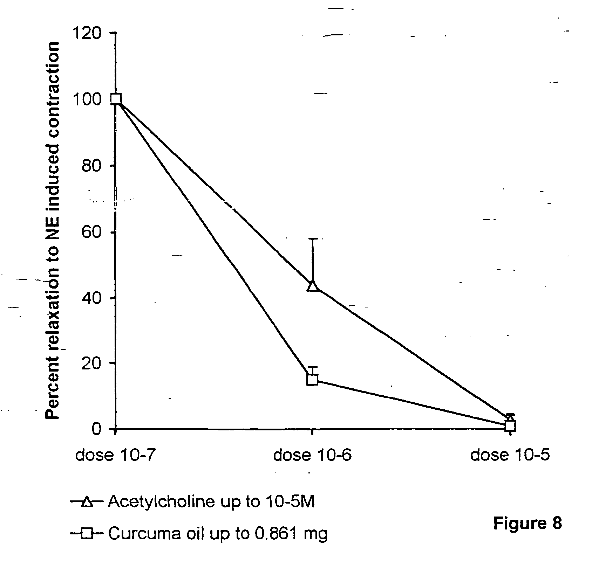

Abdominal aorta was mounted according to Wolfgang. et. al. [Wolfgang Auch-Schwelk, Zvonimir S.Katusic and Paul M.Vanhoutte : contractions to oxygen derived free radicals are augmented in aorta of the spontaneously hypertensive rats., Hypertension ,13, 859-864(1989)]. Aortic rings were contracted with norepinephrine 10⁻⁸ to 10⁻⁵ M. The contracted vessels were relaxed by acetylcholine or *Curcuma* oil, added in a stepwise manner. Acetylcholine was added in a concentration of 10⁻⁷ to 10⁻⁵ M. For *Curcuma* oil, the final contraction achieved was 0.861mg in a 8ml bath (Fig. 8). Protein Kinase C activator, Phorbol 12-Myristate 13- Acetate (PMA) (10⁻⁷ M ) induced contraction in the intact and denuded aortic strip preparation. Pretreatment with *Curcuma* oil ,0.881 mg. completely inhibited PMA induced contraction. It inhibits protein-kinase C [Kaczmarck L. K.; The role of Protein Kinase C in regulation of ion channels and neurotransmitter release: Trends in Neurosciences, 10, 30-34 (1987); Jin-Moo Lee, Grabb M.G., Zipfel G.J., Choi D. W., J. Clin. Invest., 106, (6),723-731 (2000).
Result: *Curcuma* oil and acetylcholine caused complete relaxation in norepinephrine induced contraction showing a significant vasorelaxant effect.

### Example 7

Nitric oxide was first characterized in the central nervous system at the intracellular messenger mediating the increase in cGMP levels that follows activation of glutamate receptors (Garthwaite J, Charles S.L. and Chess-Williams R. Endothelium derived relaxing factor release on activation of NMDA receptors suggest a role as intracellular messenger in the brain. Nature 336, 385-388 (1988). Simultaneous generation of NO and superoxide radicals favours the production of a toxic reaction product peroxynitrite anion (Beckman JS, Beckman TW, Chen J, Marshal PA and Freeman BA). Apparent hydroxyl radical production by peroxynitriye: implication for endothelial injury for nitric oxide and superoxide. Proc. Natl. Acad. Sci. USA 87, 1620 (1990). Once near or inside a cell peroxynitrite can damage or deplete a number of vital components, DNA by strand scission, lipid by lipid peroxidation aconitase and antioxidant availability (Cuzzocrea S, Riley. DP, Caputi AP and Salvemini D. Antioxidant therapy: a new pharmacological approach ion shock, inflammation and ischemia/reperfusion injury. Pharmacol. Rev. 53: 135-159, (2001). To test the nitric oxide scavenging property of the test agent invitro testing was carried out using an NO donor sodium nitroprusside.

### Nitric oxide (NO) scavenging by test compounds/agents:

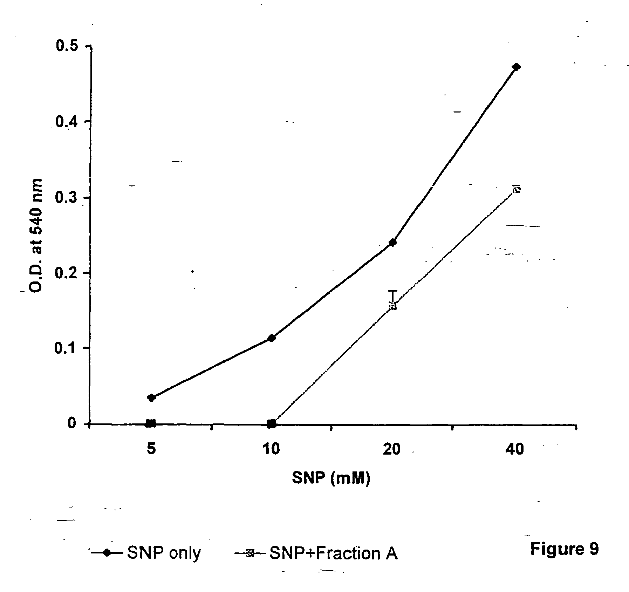

Sodium nitroprusside (SNP) generates Nitric oxide (NO) [Sreejayan and Rao M.N.A: Nitric oxide scavenging by curcuminoids, J.Pharm. Pharmacol., 49, 105-107(1997)]. fraction -A, 86.14mg was mixed in phosphate-buffer saline at different concentration of SNP ( 5-40 mM) Griess reagent in 1:1 ratio was mixed with the test compound (fraction -A). The absorbance of the above chromophore buffer formed with SNP, test compound (fraction -A) and Griess reagent was read at 546 nm and refer to the absorbance of standard solution of potassium nitrite treated in the same way with Griess reagent ( Green L.C, Wagner D.A.,Glogowski J, Skipper P.L., Wishnok J,S.,Tannenbaum S.R., Analysis of nitrate, nitrite and 15N in biological fluids; Anal. Biochem. 126 , 131(1982). Marcocci L., Maguire J.J, Droy-Lefaix M.T., Packer L.: The nitric oxide scavenging property of Ginkgo biloba extract EGb 761, Biochem. Biophys. Res. Commun. 201, 748 (1994).
Results : SNP generates nitric oxide and test compound (fraction A) scavenges the nitric oxide thus generated. The result indicated the test compound (fraction A) in focal ischaemia to be a scavenger of nitric oxide (Fig. 9).

## Claims

1. A composition obtained from a lipid soluble extract of rhizomes and/or leaves of *Curcuma species* of the Zingiberaceae family, for the treatment of neurocerebrovascular disorders, said composition comprising as its major constituents ar-d-turmerone of formula 1 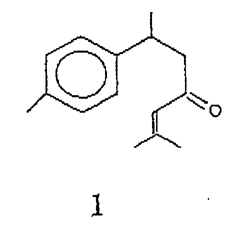 and a and 13 turmerone of formula 2, wherein in a turmerone R = -CH₃, and in β turmerone R = =CH₂ 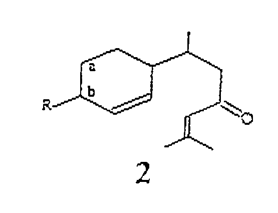 and optionally further comprising minor constituents selected from curcumene, zingiberine, germacrone, curcumerone, zedoarone, sedoarondiol, isozdedoaronidiol, curcumenone and curlone, and/or pharmaceutically acceptable additives.

2. A composition as claimed in claim 1, wherein curcuma species is *Curcuma domestica Valeton,*

3. A composition as claimed in claim 1, wherein ar-d-turmerone constitutes 95% of the composition.

4. A composition as claimed in claim 1, wherein additives are present and are selected from melatonin, antioxidants, calcium channel antagonists, tissue plasminogen activator (t-PA) and cell membrane stabilizing agents.

5. A composition as claimed in claim 1, wherein said composition inhibits nitric oxide synthase (NOS) overproduction, prevents calcium overload in neurons, and scavenges free radicals.

6. A composition as claimed in claim 1, wherein said neurocerebrovascular disorders are selected from ischaemia, stroke, post-stroke injury, hemorrhage, reperfusion injury, thrombosis, vasoconstriction, nitric oxide-induced free radical oxidative damage, infarction, inflammation and Alzheimer's disease.

7. A composition as claimed in claim 1, wherein said composition is the form of a delivery system selected from tablets, capsules, suppositories, beads and aerosols.

8. The use of a composition according to claim 1 in the manufacture of a medicament for the treatment of neurocerebrovascular disorders in animals including humans, by administration in a therapeutically effective amount.

9. The use as claimed in claim 8, wherein said use involves inhibiting nitric oxide synthase (NOS) overproduction, prevention of calcium overload in neurons, and scavenging of free radicals.

10. The use as claimed in claim 8, wherein the neurocerebrovascular disorders are selected from ischaemia, stroke, post-stroke injury, hemorrhage, reperfusion injury, thrombosis, vasoconstriction, nitric oxide-induced free radical oxidative damage, infarction, inflammation, and Alzheimer's disease.

11. The use as claimed in claim 8, wherein the effective amount ranges between 10-1000 mg/day in a divided dosage schedule.

12. The use as claimed in claim 8, wherein the said composition is administered intraperitoneally or post occlusion.

13. The use as claimed in claim 8, wherein said composition is the form of a delivery system selected from tablets, capsules, suppositories, beads, and aerosols.

14. The use as claimed in any of claims 10 to 12 wherein the neurocerebrovascular disorder is ischaemia.

15. The use as claimed in claim 14, wherein said ischaemia is severe brain ischaemia.

16. The use as claimed in claim 14, wherein said use prevents overload or calcium ions in the mitochondria.

17. The use as claimed in any of claims 14 to 12 wherein the neurocerebrovascular disorder is stroke.

18. The use as claimed in claim 17, wherein said stroke is selected from thrombotic, embolic and focal stroke.

19. The use as claimed in any of claims 10 to 12 wherein the neurocerebrovascular disorder is hemorrhage.

20. The use as claimed in claim 19, wherein the effective amount ranges between 10-500 mg/day in a divided dosage schedule.

21. The use as claimed in any of claims 14 to 12 wherein the neurocerebrovascular disorder is thrombosis.

22. The use as claimed in claim 21, wherein the thrombosis is selected from cerebral, coronary and deep vein thrombosis.

23. The use as claimed in claim 21, wherein the said use brings down the thrombus to one-fourth.

24. The use is claimed in any of claims 10 to 12 wherein the neurocerebrovascular disorder is hypertension.

25. The use as claimed in claim 24, wherein the said use brings down the blood pressure by about 40%.

26. The use as claimed inc claim 24, wherein the said use maintains the blood pressure of normotensives.

27. The use as claimed in any one of claims 10 to 12 wherein the neurocerebrovascular disorder is vasoconstriction.

28. The use as claimed in any of claims 10 to 12 wherein the neurocerebrovascular disorder is superoxide and nitric oxide-induced free radical oxidative damage.

29. The use as claimed in claim 28, wherein said use augments the level of oxygen scavenging enzymes comprising superoxide dismutase (SOD), and catalase.

30. The use as claimed in claim 28, wherein said use decreases the level of thiobarbituric acid reactive substances (TBARS).

31. The use as claimed in any of claims 10 to 12 wherein the neurocerebrovascular disorder is edema.

32. The use as claimed in claim 31, wherein said edema is selected from brain and pulmonary edema.

## Patentansprüche

1. Zusammensetzung erhalten aus einem lipidlöslichen Extrakt von Rhizomen und/oder Butter der Curcuma-Spezies der Zingiberaceae-Familie für die Behandlung von neurozerebrovaskulären Störungen, wobei die Zusammensetzung als ihre Hauptbestandteile ar-d-Tumeron der Formel 1 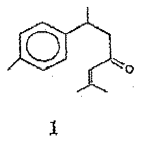 und α- und β-Turmeron der Formel 2 umfasst, wobei in α-Turmeron R=-CH₃ ist und in β-Turmeron R = =CH₂ ist 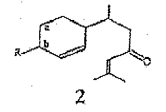 und wahlweise des Weiteren weniger bedeutende Bestandteile ausgewählt unter Curcumen, Zingiberin, Germacron, Curcumeron, Zedoaron, Sedoarondiol, Isozdedoaronidiol, Curcumenon und Curlon und/oder pharmazeutisch akzeptable Zusatzmittel umfasst.

2. Zusammensetzung nach Anspruch 1, wobei die Curcuma-Spezies *Curcuma domestica Valeton* ist.

3. Zusammensetzung nach Anspruch 1, wobei ar-d-Turmeron 95 % der Zusammensetzung ausmacht.

4. Zusammensetzung nach Anspruch 1, wobei Zusatzmittel vorliegen und unter Melatonin, Antioxidationsmitteln, Calciumkanalantagonisten, Gewebeplasminogenaktivator (t-PA) und Zellmembranen stabilisierenden Mitteln ausgewählt werden.

5. Zusammensetzung nach Anspruch 1, wobei die Zusammensetzung die Stickstoffmonoxidsynthase-(NOS)-Überproduktion inhibiert, die Calciumüberlastung in Neuronen verhindert und freie Radikale auffängt.

6. Zusammensetzung nach Anspruch 1, wobei die neurozerebrovaskulären Störungen unter Ischämie, Schlaganfall, Verletzung nach Schlaganfall, Blutung, Reperfusionsverletzung, Thrombose, Vasokonstriktion, durch Stickstoffmonoxid induziertem, durch freie Radikale hervorgerufenem Schaden, Infarkt, Entzündung und Alzheimer-Krankheit ausgewählt werden.

7. Zusammensetzung nach Anspruch 1, wobei die Zusammensetzung in Form eines unter Tabletten, Kapseln, Zäpfchen, Perlen und Aerosolen ausgewählten Abgabesystems vorlegt

8. Verwendung einer Zusammensetzung nach Anspruch 1 bei der Herstellung eines Medikaments für die Behandlung von neurozerebrovaskulären Störungen bei Tieren, einschließlich Menschen, durch Verabreichung in einer therapeutisch wirksamen Menge.

9. Verwendung nach Anspruch 8, wobei die Verwendung das Hemmen der Stickstoffmonoxidsynthase- (NOS-) Überproduktion involviert, die Calciumüberladung in Neuronen verhindert und freie Radikale auffängt.

10. Verwendung nach Anspruch 8, wobei die neurozerebrovaskulären Störungen unter Ischämie, Schlaganfall, Verletzung nach Schlaganfall, Blutung, Reperfusionsverletzung, Thrombose, Vasokonstrktion, durch Stickstoffmonoxid induziertem, durch freie Radikale hervorgerufenem Schaden, Infarkt, Entzündung und Alzheimer-Krankheit ausgewählt werden.

11. Verwendung nach Anspruch 8, wobei die wirksame Menge bei einem Behandlungsplan einzelner Dosen im Bereich zwischen 10 - 1000 mg/Tag liegt.

12. Verwendung nach Anspruch 8, wobei die Zusammensetzung intraperitoneal oder nach Okklusion verabreicht wird.

13. Verwendung nach Anspruch 8, wobei die Zusammensetzung in Form eines unter Tabletten, Kapseln, Zäpfchen, Perlen und Aerosolen ausgewählten Abgabesystems vorliegt.

14. Verwendung nach Anspruch 10 bis 12, wobei die neurozerebrovaskuläre Störung die Ischämie ist.

15. Verwendung nach Anspruch 14, wobei die Ischämie eine schwere Gehimischämie ist.

16. Verwendung nach Anspruch 14, wobei die Verwendung die Überladung der Mitochondrien mit Calciumionen verhindert.

17. Verwendung nach einem der Ansprüche 10 bis 12, wobei die neurozerebrovaskulare Störung ein Schlaganfall ist.

18. Verwendung nach Anspruch 17, wobei der Schlaganfall unter Thrombosen-, embolischem und fokalem Schlaganfall ausgewählt wird.

19. Verwendung nach einem der Ansprüche 10 bis 12, wobei die neurozerebrovaskuläre Störung eine Blutung ist.

20. Verwendung nach Anspruch 19, wobei die wirksame Menge bei einem Behandlungsplan einzelner Dosen im Bereich zwischen 10 - 500 mg/Tag liegt.

21. Verwendung nach einem der Ansprüche 10 bis 12, wobei die neurozerebrovaskuläre Störung eine Thrombose ist.

22. Verwendung nach Anspruch 21, wobei die Thrombose unter zerebraler, Herzkranzarterien- und tiefer Venenthrombose ausgewählt wird.

23. Verwendung nach Anspruch 21, wobei die Verwendung den Thrombus auf ein Viertel reduziert.

24. Verwendung nach einem der Ansprüche 10 bis 12, wobei die neurozerebrovaskuläre Störung der Bluthochdruck ist.

25. Verwendung nach Anspruch 24, wobei die Verwendung den Blutdruck um etwa 40 % reduziert.

26. Verwendung nach Anspruch 24, wobei die Verwendung den Blutdruck bei dem von Menschen mit normalem Blutdruck hält.

27. Verwendung nach einem der Ansprüche 10 bis 12, wobei die neurozerebrovaskuläre Störung die Vasokonstriktion ist.

28. Verwendung nach einem der Ansprüche 10 bis 12, wobei die neurozerebrovaskuläre Störung der durch Superoxid und Stickstoffmonoxid induzierte, durch freie Radikale hervorgerufene Schaden ist.

29. Verwendung nach Anspruch 28, wobei die Verwendung den Spiegel von Sauerstoffauffangenden Enzymen, die Superoxiddismutase (SOD) und Katalase umfassen, erhöht.

30. Verwendung nach Anspruch 28, wobei die Verwendung den Spiegel von mit Thiobarbitursäure reaktiven Substanzen (TBARS) reduziert.

31. Verwendung nach einem der Ansprüche 10 bis 12, wobei die neurozerebrovaskuläre Störung ein Ödem ist.

32. Verwendung nach Anspruch 31, wobei das Ödem unter Gehirn- und Lungenödem ausgewählt wird.

## Revendications

1. Composition obtenue à partir d'un extrait soluble dans les lipides de rhizomes et/ou de feuilles d'espèces du genre *Curcuma* de la famille des Zingibéracée, pour le traitement de troubles neurocérébrovasculaires, ladite composition comprenant en tant que constituants majeurs une ar-d-turmérone de formule 1 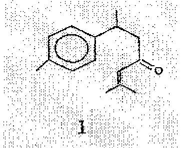 et une α- et β-turmérone de formule 2, où dans l'α-turmérone R = -CH₃, et dans la β-turmérone R = =CH₂ 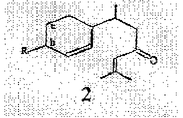 et comprenant éventuellement en outre des constituants mineurs sélectionnés parmi le curcumène, la zingibérine, la germacrone, la curcumérone, la zédoarone, le sédoarondiol, l'isozdédoaronidiol, la curcuménone et la curlone, et/ou des additifs pharmaceutiquement acceptables.

2. Composition selon la revendication 1, dans laquelle l'espèce du genre Curcuma est *Curcuma domestica Valeton.*

3. Composition selon la revendication 1, dans laquelle l'ar-d-turmérone constitue 95% de la composition.

4. Composition selon la revendication 1, dans laquelle des additifs sont présents et sont sélectionnés parmi la mélatonine, les antioxydants, les inhibiteurs des canaux calciques, l'activateur tissulaire du plasminogène (t-PA) et les agents stabilisant les membranes cellulaires.

5. Composition selon la revendication 1, laquelle composition inhibe la surproduction d'oxyde nitrique synthase (NOS), évite une surcharge en calcium dans les neurones, et piège les radicaux libres.

6. Composition selon la revendication 1, dans laquelle lesdits troubles neurocérébrovasculaires sont sélectionnés parmi l'ischémie, l'accident vasculaire cérébral (AVC), les lésions post-AVC, l'hémorragie, les lésions faisant suite à une reperfusion, la thrombose, la vasoconstriction, les dommages oxydatifs dus aux radicaux libres induits par l'oxyde nitrique, l'infarctus, l'inflammation et la maladie d'Alzheimer.

7. Composition selon la revendication 1, laquelle composition se présente sous la forme d'un système d'administration sélectionné parmi les comprimés, les capsules, les suppositoires, les billes et les aérosols.

8. Utilisation d'une composition selon la revendication 1 dans la fabrication d'un médicament pour le traitement de troubles neurocérébrovasculaires chez les animaux y compris les humains, par administration en quantité thérapeutiquement efficace.

9. Utilisation selon la revendication 8, dans laquelle ladite utilisation implique l'inhibition de la surproduction d'oxyde nitrique synthase (NOS), la prévention d'une surcharge en calcium dans les neuroxies, et le piégeage des radicaux libres.

10. Utilisation selon la revendication 8, dans laquelle les troubles neurocérébrovasculaires sont sélectionnés parmi l'ischémie, l'accident vasculaire cérébral (AVC), les lésions post-AVC, l'hémorragie, les lésions faisant suite à une reperfusion, la thrombose, la vasoconstriction, les dommages oxydatifs dus aux radicaux libres induits par l'oxyde nitrique, l'infarctus, l'inflammation et la maladie d'Alzheimer.

11. Utilisation selon la revendication 8, dans laquelle la quantité efficace est comprise entre 10 et 1000 mg/jour selon un schéma posologique fractionné.

12. Utilisation selon la revendication 8, dans laquelle ladite composition est administrée par voie intrapéritonéale ou post-occlusion.

13. Utilisation selon la revendication 8, dans laquelle ladite composition se présente sous la forme d'un système d'administration sélectionné parmi les comprimés, les capsules, les suppositoires, les billes et les aérosols.

14. Utilisation selon l'une quelconque des revendications 10 à 12 dans laquelle le trouble neurocérébrovasculaire est l'ischémie.

15. Utilisation selon la revendication 14, dans laquelle ladite ischémie est une ischémie cérébrale sévère.

16. Utilisation selon la revendication 14, laquelle utilisation évite une surcharge d'ions calcium dans les mitochondries,

17. Utilisation selon l'une quelconque des revendications 10 à 12 dans laquelle le trouble neurocérébrovasculaire est l'accident vasculaire cérébral (AVC).

18. Utilisation selon la revendication 17, dans laquelle ledit AVC est sélectionné parmi l'AVC thrombotique, l'AVC embolique et l'AVC focal.

19. Utilisation selon l'une quelconque des revendications 10 à 12 dans laquelle le trouble neurocérébrovasculaire est l'hémorragie.

20. Utilisation selon la revendication 19, dans laquelle la quantité efficace est comprise entre 10 et 500 mg/jour selon un schéma posologique fractionné.

21. Utilisation selon l'une quelconque des revendications 10 à 12 dans laquelle le trouble neurocérébrovasculaire est la thrombose.

22. Utilisation selon la revendication 21, dans laquelle la thrombose est sélectionnée parmi la thrombose cérébrale, la thrombose coronaire et la thrombose veineuse profonde.

23. Utilisation selon la revendication 21, laquelle utilisation réduit le thrombus à un quart.

24. Utilisation selon l'une quelconque des revendications 10 à 12 dans laquelle le trouble neurocérébrovasculaire est l'hypertension,

25. Utilisation selon la revendication 24, laquelle utilisation fait baisser la pression sanguine d'environ 40%,

26. Utilisation selon la revendication 24, laquelle utilisation maintient la pression sanguine des sujets normotendus.

27. Utilisation selon l'une quelconque des revendications 10 à 12 dans laquelle le trouble neurocérébrovasculaire est la vasoconstriction.

28. Utilisation selon l'une quelconque des revendications 10 à 12 dans laquelle le trouble neurocérébrovasculaire consiste en dommages oxydatifs dus aux radicaux libres induits par le radical superoxyde et l'oxyde nitrique.

29. Utilisation selon la revendication 28, laquelle utilisation augmente le taux des enzymes piégeant l'oxygène comprenant la superoxyde dismutase (SOD) et la catalase.

30. Utilisation selon la revendication 28, laquelle utilisation diminue le taux des substances réagissant avec l'acide thiobarbiturique (les TBARS).

31. Utilisation selon l'une quelconque des revendications 10 à 12 dans laquelle le trouble neurocérébrovasculaire est l'oedème.

32. Utilisation selon la revendication 31, dans laquelle ledit oedème est sélectionné parmi l'oedème cérébral et l'oedème pulmonaire.
